# DEMANDE DE BREVET EUROPEEN

(11) **EP 1 702 563 A2**
(43) Date de publication de la demande: **20.09.2006**
(21) Numéro de dépôt: 06300232.3
(22) Date de dépôt: 14.03.2006
(51) Int. Cl.: A61B 5/103

(54) **Dispositif de mesure du teint**

(30) Priorité: 15.03.2005 FR 0550667
(71) Demandeur: L'ORÉAL, 75008 Paris (FR)
(72) Inventeur: LEVEQUE, Jean-Luc, 75017, PARIS (FR); BAZIN, Roland, 91570, BIEVRES (FR)
(74) Mandataire: Tanty, François

(57) **Abrégé**

La présente invention concerne un dispositif d'évaluation du teint, comportant :
- une tête de mesure à appliquer contre la peau, cette tête comportant :
   - une fenêtre de sortie (5) par laquelle une lumière est émise dans la peau,
   - une fenêtre de détection (6) pour recevoir la lumière émise par la fenêtre de sortie, la distance (*d*) entre les fenêtres de sortie et de détection étant choisie de manière à ce qu'au moins la majeure partie de la lumière émise par la fenêtre de sortie (5) parcoure au sein de la peau une distance d'au moins 1 mm, avant d'atteindre la fenêtre de détection (6),
- un système de détection non colorimétrique délivrant un signal représentatif de l'intensité lumineuse captée par la fenêtre de détection.

## Description

La présente invention concerne les dispositifs et procédés permettant d'évaluer des caractéristiques physiques et biologiques de la peau.

Le brevet européen EP 0 655 221 B1 décrit un procédé permettant de déterminer la couleur interne de la peau, dans lequel on applique contre celle-ci une tête de mesure colorimétrique. La lumière qui ressort de la peau après diffusion dans ses couches profondes est analysée colorimétriquement.

La demande internationale WO 88/05284 décrit un appareil pour déterminer un indice de protection solaire, comportant une diode luminescente émettant une lumière verte et une cellule photovoltaïque. La position de cette cellule est choisie de telle sorte que presque seule la lumière réfléchie par la couche mélanique soit captée par la cellule.

La demande DE 40 31 320 décrit un dispositif comportant une tête de mesure à appliquer sur la peau, pour analyser la distribution spectrale d'une lumière diffusée par la peau.

La demande de brevet FR 2 658 410 décrit un appareil pour mesurer le degré de coloration de la peau, comportant une cellule photo-électrique agencée pour recevoir un faisceau lumineux réfléchi par la peau.

La demande de brevet GB 2 022 244 décrit un dispositif pour l'étude des jaunisses, comportant un récepteur de lumière pourvu de filtres bleu et vert.

Il existe un besoin pour bénéficier d'un moyen permettant d'évaluer le teint de la peau.

Le terme « teint » englobe ici plus que la notion de couleur en y ajoutant celle de comportement face à la lumière incidente.

Le teint est ainsi fonction de l'irrigation sanguine de la peau et de la densité des follicules pileux, lesquels se comportent en quelque sorte comme des pièges à lumière vis-à-vis de la lumière incidente.

L'invention a pour objet, selon l'un de ses aspects parmi d'autres, un dispositif d'évaluation du teint, comportant :
- une tête de mesure à appliquer contre la peau, cette tête comportant :
   - une fenêtre de sortie par laquelle une lumière, par exemple une lumière visible, peut être émise dans la peau,
   - une fenêtre de détection pour recevoir la lumière émise par la fenêtre de sortie, la distance entre les fenêtres de sortie et de détection étant de préférence choisie de manière à ce qu'au moins la majeure partie de la lumière émise par la fenêtre de sortie parcoure au sein de la peau au moins 1 mm, mieux au moins 3 ou 5 mm, mieux encore au moins 7 mm avant d'atteindre la fenêtre de détection,
- un système de détection non colorimétrique délivrant un signal représentatif de l'intensité lumineuse captée par la fenêtre de détection.

La tête de mesure peut être agencée de manière à créer au moins deux trajets de lumière de longueur et/ou d'orientation différentes dans la peau.

La distance parcourue au sein de la peau est par exemple comprise entre 1 et 10 mm.

On entend par « système de détection non colorimétrique » un système de détection délivrant des informations ne permettant pas de déterminer les coordonnées dans un espace colorimétrique de la lumière captée par la fenêtre de détection.

En particulier, le système de détection non colorimétrique peut être dépourvu de moyen d'analyse d'un spectre de réflectance.

Le terme « fenêtre » ne doit pas être compris avec un sens restrictif et englobe toute région d'émission ou de détection de la lumière. Une fenêtre peut être définie par une ouverture à travers une paroi du dispositif mais il peut en être différemment.

Lorsque la tête de mesure est appliquée sur la peau, la fenêtre de détection ne reçoit que la lumière ayant diffusé dans la peau et provenant de la fenêtre de sortie. Cette lumière se propage sur un trajet suffisamment long dans les couches profondes de la peau pour que l'intensité détectée varie de manière significative entre des individus considérés comme n'ayant pas le même teint.

L'intensité lumineuse mesurée peut dépendre notamment de la densité plus ou moins grande de follicules pileux, une densité élevée de follicules s'accompagnant généralement également d'une irrigation sanguine plus grande.

De plus l'invention, de par l'utilisation d'un système de détection non colorimétrique, est simple à réaliser et de fonctionnement fiable.

La distance entre les fenêtres de sortie et de détection, lorsqu'elle est relativement importante, peut permettre de prendre en compte l'influence sur le teint des couches relativement profondes de la peau.

Le dispositif peut comporter une source lumineuse pouvant être une source de lumière blanche, par exemple une ou plusieurs ampoules à incandescence ou diodes électroluminescentes blanches. La lumière émise par la fenêtre de sortie peut, en variante, être dans le domaine infrarouge ou ultraviolet.

Le dispositif peut comporter ou non, selon les besoins, au moins un filtre optique agencé pour filtrer la lumière captée par la fenêtre de détection, par exemple être un filtre vert, jaune ou rouge.

Le dispositif peut comporter au moins un monochromateur ou un filtre en émission et/ou en réception.

La lumière envoyée dans la peau peut être sensiblement monochromatique, grâce par exemple à l'emploi d'un filtre, d'un monochromateur ou d'une source lumineuse monochromatique.

En variante, la lumière détectée peut être monochromatique tandis que la lumière émise ne l'est pas, grâce par exemple à l'emploi d'un filtre ou d'un monochromateur en réception.

De préférence, la source lumineuse est modulée en intensité, ce qui permet par l'utilisation d'une détection synchrone de s'affranchir de la luminosité ambiante.

La tête de mesure peut comporter tout capteur sensible à la lumière, visible ou non, par exemple une cellule photovoltaïque, un phototransistor, une photodiode ou un capteur linéaire ou matriciel capable de délivrer un signal électrique fonction de l'intensité lumineuse captée.

L'utilisation d'un capteur linéaire ou matriciel peut permettre d'obtenir une information sur la manière dont l'intensité lumineuse reçue décroît en fonction de la distance.

Le système de détection peut délivrer un signal numérique ou analogique.

Le système de détection peut par exemple délivrer une tension ou une intensité électrique variant en fonction de la lumière reçue par la fenêtre de détection, cette tension ou cette intensité étant par exemple transformée en une information alphanumérique lue sur un afficheur.

Le système de détection peut comporter des moyens de calibration permettant de procéder à un étalonnage préalable et peut comporter par exemple des moyens de réglage du zéro et/ou du gain. Le système de détection peut être calibré en utilisant un bloc d'un matériau présentant des propriétés optiques prédéfinies, par exemple un bloc de mousse de matière plastique, par exemple de polyéthylène, notamment celle commercialisée sous la dénomination TRESYLENE.

Dans un exemple de mise en oeuvre de l'invention, le dispositif de mesure tient tout entier dans une main, étant par exemple alimenté par au moins une pile ou une batterie électrique.

Le dispositif de mesure peut être autonome, comportant par exemple un afficheur ou tout autre moyen permettant de diffuser l'information, par exemple un cadran à aiguilles, un afficheur LCD ou à diodes électroluminescentes, un écran LCD, OLED, à plasma ou autres et/ou un ou plusieurs voyants.

Le dispositif peut comporter un moyen d'affichage permettant de délivrer une information appréhendable renseignant directement l'utilisateur sur le résultat de l'évaluation du teint, par exemple un score ou un message alphanumérique plus complexe.

Le dispositif de mesure peut encore être agencé pour transmettre à ou échanger des données avec un terminal, par exemple un ordinateur, un assistant numérique ou un téléphone mobile. L'échange de données peut s'effectuer par une liaison filaire ou non. Le terminal peut alors exécuter un programme permettant de traiter les signaux provenant du dispositif de mesure.

Le cas échéant, le dispositif de mesure peut comporter au moins un autre capteur pour mesurer une autre grandeur physico-chimique de la peau, par exemple une température, un taux d'humidité, de sébum, un biocapteur, etc.

Le dispositif de mesure peut comporter également un capteur de pression ou tout autre moyen permettant d'appliquer la tête de mesure contre la peau avec une pression suffisante pour en chasser l'hémoglobine.

La tête de mesure est par exemple montée sur un système de rappel élastique permettant de l'appliquer contre la peau avec une pression prédéfmie.

L'invention a encore pour objet, selon un autre de ses aspects, indépendamment ou en combinaison avec ce qui précède, un dispositif comportant une tête de mesure destinée à être appliquée contre la peau, cette tête de mesure étant agencée de manière à créer au moins deux trajets de lumière de longueurs et/ou d'orientations différentes dans la peau, ce dispositif comportant en outre un système de détection agencé pour mesurer des intensités lumineuses correspondantes.

Le système de détection peut permettre, le cas échéant, de connaître la couleur de la lumière ayant traversé la peau ou l'atténuation de la lumière en fonction de la longueur d'onde.

Les deux trajets de lumière sont par exemple créés avec au moins trois fenêtres.

Le dispositif comporte par exemple une fenêtre de sortie et deux fenêtres de détection situées à des distances différentes de la fenêtre de sortie et/ou avec des orientations angulaires différentes par rapport à la fenêtre de sortie.

Le dispositif peut encore comporter au moins une source lumineuse et un capteur matriciel et/ou linéaire permettant de détecter l'intensité et/ou la couleur de la lumière après deux trajets de longueurs et/ou d'orientations différentes dans la peau.

Le dispositif peut comporter des moyens de traitement pour délivrer, en fonction des intensités lumineuses associées aux différents trajets, au moins une information permettant de comparer les propriétés optiques de la peau à des profondeurs différentes.

La détection de l'intensité captée s'effectue par exemple en parallèle pour chacune des fenêtres de détection ou en variante de manière séquentielle.

Les deux trajets de lumière peuvent être sensiblement orthogonaux ou colinéaires ou encore former entre eux un angle prédéfmi.

Dans un exemple de mise en oeuvre de l'invention, le dispositif comporte une fenêtre de sortie et une pluralité de fenêtres de détection disposées équi-angulairement autour de la fenêtre de sortie, à distance constante de celle-ci ou non.

Les fenêtres de détection peuvent par exemple être au nombre de trois ou plus, étant par exemple au nombre de douze avec un angle de 30° entre deux fenêtres de détection voisines.

L'invention a encore pour objet, selon un autre de ses aspects, un procédé d'évaluation de propriétés physiques et biologiques de la peau, notamment du teint, dans lequel on utilise un dispositif tel que défini plus haut, comportant deux fenêtres ou plus, la tête de mesure étant appliquée contre la peau de manière à en chasser l'hémoglobine au moment de la mesure.

Deux mesures effectuées dans deux régions différentes du corps peuvent mettre en évidence par exemple une différence de teint entre celles-ci et le besoin d'appliquer localement sur l'une d'elle un produit visant par exemple à agir sur la microcirculation sanguine, la sécrétion de sébum, ou l'épaisseur de la peau (épiderme ou derme).

L'invention a encore pour objet un procédé d'évaluation de propriétés physiques et biologiques de la peau, notamment du teint, dans lequel on mesure l'atténuation de la lumière dans la peau selon deux trajets de longueurs et/ou d'orientation différentes.

Lorsque le dispositif utilisé ne comporte que deux fenêtres de mesure, on peut modifier l'orientation du dispositif entre les mesures.

Lorsque le dispositif utilisé est agencé de manière à créer des trajets de lumière dans la peau non colinéaires, par exemple sensiblement orthogonaux, et comporte par exemple au moins trois fenêtres à cet effet, des mesures de l'atténuation de la lumière selon ces deux trajets peuvent être effectuées sans qu'il n'y ait besoin de déplacer le dispositif ou de modifier son orientation entre les mesures.

Le dispositif peut comporter par exemple des moyens de traitement permettant par exemple de comparer au moins deux valeurs résultant de mesures effectuées suivant au moins deux orientations relativement à la peau, par exemple deux orientations sensiblement perpendiculaires entre elles ou plusieurs orientations espacées régulièrement.

Cela peut permettre par exemple de mettre en évidence l'orientation des faisceaux de collagène dans la peau et d'en déduire une information concernant par exemple l'état de vieillissement de la peau.

L'invention a encore pour objet un procédé pour mettre en évidence l'effet d'un traitement sur des caractéristiques physiques et biologiques de la peau, notamment le teint, par exemple l'application d'un produit ou la prise d'un complément alimentaire, dans lequel on effectue au moins deux évaluations de la peau, avant et après le traitement, en utilisant le dispositif tel que défini plus haut. Le traitement peut être un traitement médical ou non médical. Le traitement peut, le cas échéant, comporter la prise ou l'application d'un médicament.

On peut par exemple attribuer à chaque mesure une valeur, notamment numérique, et comparer les valeurs avant et après traitement.

Le cas échéant, on peut comparer la valeur obtenue avec celle rencontrée dans une population de référence, cette population de référence étant par exemple une population dont les individus présentent des caractéristiques physiologiques et/ou de typologie corporelle comparables avec celles de l'individu objet de la mesure.

L'invention a encore pour objet un procédé de prescription d'un traitement, dans lequel on évalue le teint en utilisant un dispositif tel que défini plus haut, et l'on prescrit un traitement en fonction de la mesure effectuée. Il est par exemple possible de sélectionner un traitement tel que l'application d'un produit ou la prise d'un complément alimentaire au vu de l'évaluation du teint.

L'invention a encore pour objet un procédé pour formuler un produit personnalisé, cosmétique ou dermatologique, dans lequel on évalue le teint en utilisant un dispositif tel que défini plus haut, puis en fonction des informations ainsi obtenues, la teneur d'au moins un composé entrant dans la formulation du produit est déterminée.

L'invention a encore pour objet un procédé pour promouvoir la vente d'un produit cosmétique, dans lequel il est fait état d'une action du produit sur le teint mise en évidence par l'utilisation d'un dispositif tel que défmi plus haut.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 représente de manière schématique, en perspective, un exemple de dispositif d'évaluation du teint réalisé conformément à l'invention,
- la figure 2 est une vue de face du dispositif selon la flèche II de la figure 1,
- la figure 3 représente de manière schématique le système de détection,
- la figure 4 illustre la diffusion de la lumière dans la peau,
- la figure 5 représente de manière schématique une variante du dispositif d'évaluation, comportant un ressort,
- la figure 6 illustre la possibilité de réaliser un dispositif d'évaluation pouvant communiquer avec un terminal,
- les figures 7 et 8 sont deux vues analogues à la figure 2 de deux variantes de réalisation,
- la figure 9 est une vue analogue à la figure 3 d'une variante de réalisation, et
- les figures 10 et 11 sont deux autres vues analogues à la figure 2, de variantes de réalisation.

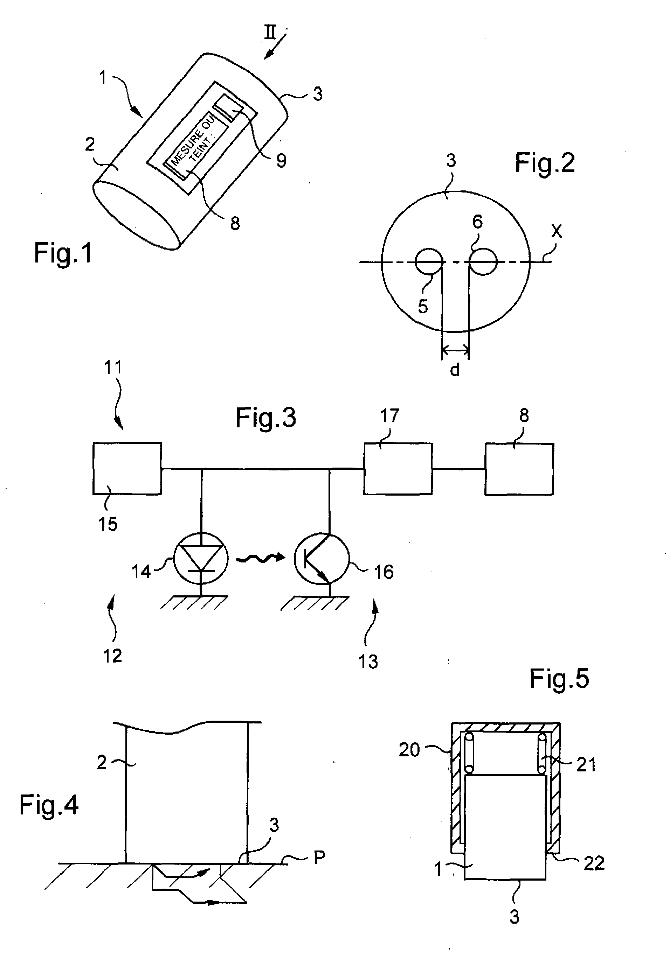

Le dispositif d'évaluation du teint 1 représenté à la figure 1 comporte un boîtier 2 dont une face 3 sert de tête de mesure en étant appliquée contre la peau.

La tête de mesure comporte une fenêtre de sortie 5 à travers laquelle une lumière est émise vers la peau et une fenêtre de détection 6 pour recueillir la lumière ayant diffusé dans la peau.

Dans l'exemple considéré, la distance d entre les fenêtres 5 et 6 est supérieure à 5 mm, étant par exemple de l'ordre de 8 mm.

Le boîtier 2 peut loger une alimentation électrique autonome telle que par exemple au moins une pile ou une batterie et peut être muni également d'un afficheur 8 permettant de délivrer une information visuelle relative à la mesure effectuée, par exemple une valeur numérique quantifiant le teint ou une grandeur associée à celui-ci.

Le dispositif 1 peut également comporter un ou plusieurs boutons de commande 9 permettant sa mise en marche et, le cas échéant, sa calibration, par exemple le réglage du zéro et du gain. Cette calibration peut s'effectuer par exemple en faisant diffuser la lumière entre les fenêtres 5 et 6 dans une matière donnée, par exemple un bloc de matière plastique connue sous la dénomination TRESYLENE.

Le dispositif 1 comporte un système de détection 11 que l'on a représenté de manière schématique à la figure 3.

Ce système de détection 11 comporte une partie émettrice 12 et une partie réceptrice 13. La partie émettrice 12 comporte par exemple une diode électroluminescente 14, de préférence émettant une lumière blanche, et un circuit d'alimentation de cette diode 14, par exemple avec une intensité modulée à une fréquence constante.

La partie réceptrice 13 comporte un capteur 16 sensible à la lumière, par exemple un phototransistor ou une photodiode, et un circuit de mesure 17 relié d'une part au capteur 16 et d'autre part à la partie émettrice 15, de manière à effectuer une détection synchrone.

Le circuit de mesure 17 comporte des moyens de traitement du signal permettant par exemple de provoquer l'affichage sur l'afficheur 8 d'une valeur numérique représentative de l'intensité lumineuse détectée par le capteur 16.

Pour utiliser le dispositif 1, l'utilisateur peut appuyer la face 3 contre la peau P, comme illustré à la figure 4.

La lumière émise par la diode 14 traverse la fenêtre de sortie 5 et diffuse dans la peau. La lumière diffusée est détectée par le capteur 16 à travers la fenêtre de détection 6. Aucune lumière émise par la fenêtre 5 ne gagne directement la fenêtre 6 sans s'être propagée dans la peau.

De préférence, le dispositif 1 est pressé contre la peau avec une pression suffisante pour en chasser l'hémoglobine, cette pression étant par exemple d'environ 1 kg/cm² ou plus.

Le cas échéant, le dispositif 1 comporte un capteur de pression et le système de détection est agencé pour effectuer la mesure et/ou informer l'utilisateur lorsqu'une pression suffisante est exercée sur la peau, par exemple par affichage d'un message correspondant sur l'afficheur 8.

Dans une variante, le dispositif 1 comporte des moyens permettant d'appliquer la face 3 avec une force prédéfmie sur la peau.

Dans l'exemple de la figure 5, le dispositif 1 est engagé dans un boîtier extérieur 20 dans lequel est disposé un ressort 21 qui est comprimé par le dispositif 1 lorsque celui-ci est appuyé sur la peau.

Lors de la mesure, la face 3 est par exemple appliquée sur la peau jusqu'à ce que le bord inférieur 22 du boîtier 20 vienne en appui contre la peau. Le ressort est alors comprimé d'une course prédéfinie, ce qui permet l'application d'une pression contrôlée sur la peau.

L'information délivrée par l'afficheur 8 est par exemple comparée avec une valeur correspondante d'une population de référence.

Le dispositif 1 peut être utilisé pour mettre en évidence une anisotropie de la propagation de la lumière dans la peau. Le dispositif peut par exemple comporter des moyens de traitement permettant de comparer les résultats de mesures effectuées avec deux orientations différentes des fenêtres 5 et 6 relativement à la peau.

Le dispositif peut comporter plus de deux fenêtres de mesure.

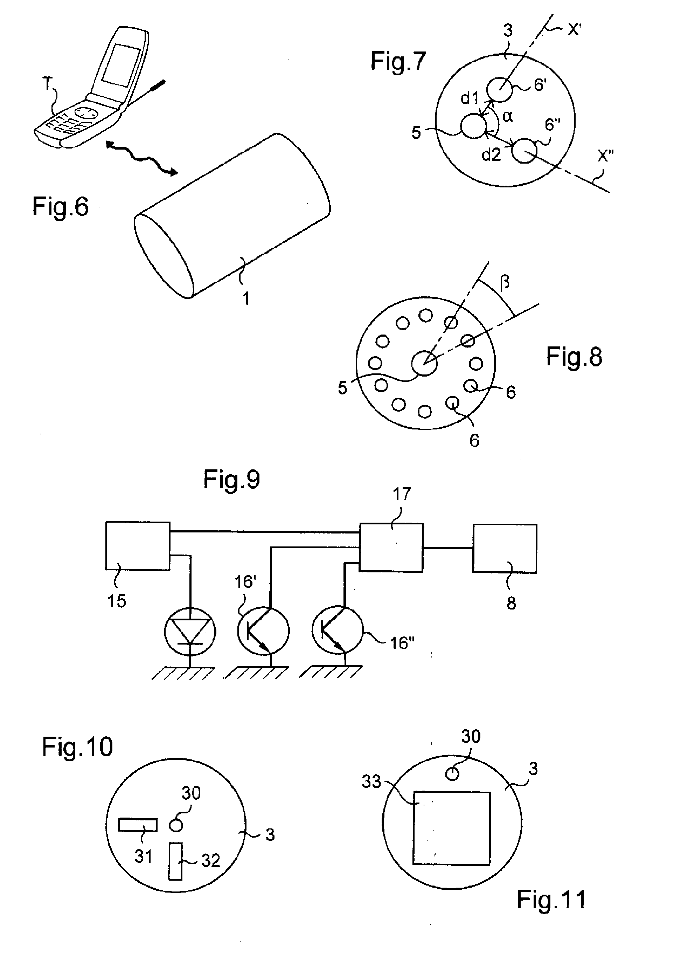

On a représenté sur les figures 7 et 9 une variante de réalisation dans laquelle la face 3 comporte une fenêtre de sortie 5 et deux fenêtres de détection 6' et 6", associées à des capteurs respectifs 16' et 16".

La distance d₁ entre les fenêtres 5 et 6' est différente de la distance d₂ entre les fenêtres 5 et 6", de manière à créer deux trajets de lumière de longueurs différentes dans la peau.

Cela peut permettre par exemple de comparer les propriétés optiques de la peau à des profondeurs différentes.

La détection de l'intensité lumineuse captée par les fenêtres 6' et 6" peut s'effectuer simultanément ou séquentiellement. Une détection séquentielle peut, le cas échéant, simplifier l'électronique de mesure.

L'angle α entre les axes X' et X" définis par les fenêtres 5 et 6' d'une part et 5 et 6" d'autre part, est par exemple égal à 0 ou à 90°.

Lorsque l'angle α est nul, les fenêtres 5, 6' et 6" sont alignées. On peut par exemple avoir d₂/d₁ compris entre 2 et 10, avec par exemple d₁= 1 mm et d₂= 5 mm.

Lorsque l'angle α est non nul, étant par exemple de 90°, on a par exemple d₁ égal à d₂ de manière à pouvoir comparer l'atténuation de la lumière dans des directions différentes, par exemple deux directions orthogonales, et ainsi obtenir une information sur l'anisotropie de la peau, notamment sur l'état du collagène et son orientation.

La tête de mesure peut encore comporter plus de trois fenêtres, par exemple deux fenêtres de sortie et deux fenêtres de détection.

Le dispositif peut comporter deux fenêtres de sortie et une fenêtre de détection, les deux fenêtres de sortie émettant alors successivement la lumière captée par la fenêtre de détection.

Le dispositif peut comporter, en variante, une fenêtre de sortie 5 et plusieurs, par exemple douze, fenêtres de détection 6, comme illustré sur la figure 8.

Les fenêtres de détection 6 sont disposées équi-angulairement autour de la fenêtre de sortie 5, avec par exemple un angle β de 30° entre deux fenêtres de détection 6 voisines.

Cette configuration permet d'effectuer des mesures suivant plusieurs orientations différentes.

Dans l'exemple de la figure 10, on a illustré la possibilité d'utiliser au moins une source lumineuse 30 et au moins un capteur linéaire 31. Ce capteur comporte une succession de cellules de détection juxtaposées et peut permettre l'analyse d'un profil d'intensité lumineuse en fonction de la distance à la source 30.

Le cas échéant, comme illustré, le dispositif comporte au moins un deuxième capteur linéaire 32, de manière à analyser l'intensité de la lumière s'étant propagée dans une direction perpendiculaire à l'orientation du capteur 31.

Dans l'exemple de la figure 11, le dispositif comporte au moins une source 30 et un capteur matriciel 33, par exemple de type CMOS ou CCD, de résolution par exemple 512 x 512.

Le dispositif peut comporter des moyens d'analyse, non représentés, reliés au capteur 33 pour analyser par exemple l'intensité lumineuse en fonction de la distance à la source 30, dans une ou plusieurs directions.

Bien entendu, l'invention n'est pas limitée aux exemples qui viennent d'être donnés.

L'invention n'est notamment pas limitée à un moyen particulier d'affichage de l'information délivrée par le dispositif 1.

Dans la variante illustrée à la figure 6, le dispositif 1 en lui-même ne comporte plus l'afficheur 8 et l'information est transmise, par exemple par une liaison filaire ou sans fil, à un terminal T, par exemple un téléphone mobile dont l'afficheur est utilisé pour informer l'utilisateur de la valeur mesurée ou un ordinateur.

Le terminal T peut effectuer au moins une partie du traitement de l'information.

Le cas échéant, l'information recueillie par le terminal T est également transmise à un site distant où elle peut être traitée et l'utilisateur peut recevoir en retour un conseil, par exemple sous la forme d'une prescription, ou un produit ayant un effet sur le teint.

Le dispositif 1 peut encore être utilisé par exemple pour mesurer l'effet d'un traitement sur le teint de la peau.

Une première mesure peut être effectuée avant le traitement, puis une deuxième mesure est effectuée après le traitement. La comparaison des valeurs délivrées par le dispositif avant et après traitement peut permettre de déterminer l'efficacité de celui-ci.

Le dispositif 1 peut être utilisé sur un point de vente ou à domicile.

Dans le cas d'une utilisation sur un point de vente, le consommateur peut, par exemple après mesure de son teint, recevoir une information concernant un produit à appliquer ou un traitement à effectuer, par exemple la prise de compléments alimentaires.

Le cas échéant, un produit personnalisé peut être formulé sur le point de vente au vu du résultat de la mesure ou envoyé ensuite au consommateur à son domicile.

Le dispositif 1 peut comporter ou être associé à au moins un capteur additionnel, par exemple un capteur d'hydratation de la peau ou du microrelief de celle-ci, ainsi que des moyens de traitement des signaux délivrés par ce ou ces capteurs additionnels, afin de renseigner l'utilisateur sur son teint et sur d'autres caractéristiques de sa peau en fonction des informations en provenance des différents capteurs.

La détection de la lumière peut s'effectuer de manière non synchrone, le cas échéant.

L'expression « comportant un » doit être comprise comme étant synonyme de « comportant au moins un », sauf si le contraire est spécifié.

## Revendications

1. Dispositif (1) d'évaluation du teint, comportant :
- une tête de mesure à appliquer contre la peau, cette tête comportant :
- une fenêtre de sortie (5) par laquelle une lumière est émise dans la peau,
- une fenêtre de détection (6) pour recevoir la lumière émise par la fenêtre de sortie, la distance (*d*) entre les fenêtres de sortie et de détection étant choisie de manière à ce qu'au moins la majeure partie de la lumière émise par la fenêtre de sortie (5) parcoure au sein de la peau une distance d'au moins 1 mm, avant d'atteindre la fenêtre de détection (6),
- un système de détection (11) non colorimétrique délivrant un signal représentatif de l'intensité lumineuse captée par la fenêtre de détection.

2. Dispositif selon la revendication 1, **caractérisé par le fait que** la distance (d) entre les fenêtres de sortie et de détection est choisie de telle sorte qu'au moins la majeure partie de la lumière émise par la fenêtre de sortie parcoure au sein de la peau une distance d'au moins 3 mm.

3. Dispositif selon la revendication 1, **caractérisé par le fait que** la distance (d) entre les fenêtres de sortie et de détection est choisie de telle sorte qu'au moins la majeure partie de la lumière émise par la fenêtre de sortie parcoure au sein de la peau une distance d'au moins 5 mm.

4. Dispositif selon la revendication 1, **caractérisé par le fait que** la distance (d) entre les fenêtres de sortie et de détection est choisie de telle sorte qu'au moins la majeure partie de la lumière émise par la fenêtre de sortie parcoure au sein de la peau une distance d'au moins 7 mm.

5. Dispositif selon l'une quelconque des revendications 1 à 4, **caractérisé par le fait que** le système de détection () est dépourvu de moyen d'analyse d'un spectre de réflectance.

6. Dispositif selon l'une des revendications précédentes, **caractérisé par le fait qu'**il comporte une source de lumière blanche (14).

7. Dispositif selon l'une quelconque des revendications 1 à 5, **caractérisé par le fait que** la lumière émise par la fenêtre de sortie et détectée est une lumière infrarouge.

8. Dispositif selon l'une quelconque des revendications 1 à 5, **caractérisé par le fait que** la lumière émise par la fenêtre de sortie et détectée est une lumière ultraviolette.

9. Dispositif selon l'une quelconque des revendications précédentes, **caractérisé par le fait qu'**il comporte au moins un filtre pour filtrer la lumière captée par la fenêtre de détection.

10. Dispositif selon l'une quelconque des revendications précédentes, **caractérisé par le fait qu'**il comporte une source lumineuse modulée en intensité.

11. Dispositif selon l'une quelconque des revendications précédentes, **caractérisé par le fait que** la tête de mesure comporte un capteur (16) sensible à la lumière, notamment un phototransistor, une photodiode (14), un capteur linéaire (31) ou matriciel (33).

12. Dispositif selon l'une quelconque des revendications précédentes, **caractérisé par le fait que** le système de détection (11) comporte des moyens de calibration permettant de procéder à un étalonnage préalable.

13. Dispositif selon l'une quelconque des revendications précédentes, **caractérisé par le fait qu'**il comporte un moyen (20, 21) permettant d'appliquer la tête de mesure contre la peau avec une pression prédéfinie.

14. Dispositif selon l'une quelconque des revendications précédentes, **caractérisé par le fait qu'**il est agencé pour tenir tout entier dans une main.

15. Dispositif selon l'une quelconque des revendications précédentes, **caractérisé par le fait qu'**il est agencé pour transmettre à ou échanger des données avec un terminal (T) tel qu'un ordinateur, un assistant numérique ou un téléphone mobile.

16. Dispositif selon l'une quelconque des revendications précédentes, **caractérisé par le fait qu'**il comporte des moyens de traitement (17) permettant de comparer deux valeurs résultant de mesures effectuées suivant deux orientations relativement à la peau.

17. Dispositif selon l'une quelconque des revendications précédentes, **caractérisé par le fait qu'**il est agencé de manière à créer au moins deux trajets de lumière de longueurs et/ou d'orientations différentes dans la peau et mesurer des intensités lumineuses correspondantes.

18. Dispositif selon la revendication précédente, **caractérisé par le fait qu'**il comporte une fenêtre de sortie (5) et deux fenêtres de détection (6', 6") situées à des distances différentes de la fenêtre de sortie.

19. Dispositif selon l'une des revendications 13 et 14, **caractérisé par le fait qu'**il comporte une fenêtre de sortie (5) et une pluralité de fenêtres de détection (6) disposées équi-angulairement autour de la fenêtre de sortie (5).

20. Dispositif selon la revendication 19, **caractérisé par le fait que** les fenêtres de détection sont disposées à une distance constante de la fenêtre de sortie, notamment à une distance constante de celle-ci (5).

21. Dispositif selon l'une des revendications 17 à 20, **caractérisé par le fait qu'**il comporte des moyens de traitement pour délivrer une information sur au moins une propriété de la peau suivant la profondeur et/ou l'orientation, en fonction des intensités lumineuses associées aux différents trajets.

22. Dispositif selon l'une quelconque des trois revendications immédiatement précédentes, **caractérisé par le fait que** la détection de l'intensité captée s'effectue en parallèle pour chacune des fenêtres de détection (6, 6").

23. Dispositif selon l'une quelconque des revendications 17 à 22, **caractérisé par le fait que** la détection de l'intensité captée s'effectue de manière séquentielle.

24. Dispositif selon l'une quelconque des revendications 17 à 23, **caractérisé par** le fait les deux trajets de lumière sont sensiblement orthogonaux.

25. Dispositif comportant une tête de mesure destinée à être appliquée contre la peau, cette tête de mesure étant agencée de manière à créer au moins deux trajets de lumière de longueurs et/ou d'orientations différentes dans la peau, ce dispositif comportant en outre un système de détection agencé pour mesurer des intensités lumineuses correspondantes.

26. Dispositif selon la revendication 25, dans lequel les deux trajets sont sensiblement colinéaires.

27. Dispositif selon la revendication 25, dans lequel les deux trajets sont sensiblement orthogonaux.

28. Dispositif selon la revendication 25, **caractérisé par le fait qu'**il comporte un capteur linéaire permettant d'analyser l'intensité lumineuse reçue à différentes distances de la source.

29. Dispositif selon la revendication 25, **caractérisé par le fait qu'**il comporte un capteur matriciel permettant d'analyser l'intensité lumineuse reçue à différentes distances de la source et/ou dans différentes directions.

30. Procédé de mesure du teint, dans lequel on utilise un dispositif comportant :
- une tête de mesure à appliquer contre la peau, cette tête comportant :
• une fenêtre de sortie (5) par laquelle une lumière est émise,
• une fenêtre de détection (6) pour recevoir la lumière émise par la fenêtre de sortie,
- un système de détection (11) non colorimétrique de la lumière rentrant dans la fenêtre de détection, ce système étant agencé pour délivrer un signal représentatif de l'intensité lumineuse captée par la fenêtre de détection,
la tête de mesure étant appliquée contre la peau de manière à en chasser l'hémoglobine au moment de la mesure.

31. Procédé selon la revendication précédente, **caractérisé par le fait que** la tête de mesure est appliquée sur la peau avec une pression d'environ 1 kg/cm².

32. Procédé pour mettre en évidence l'effet sur le teint d'un traitement cosmétique, dans lequel on effectue au moins deux évaluations du teint avant et après le traitement, en utilisant un dispositif de mesure du teint tel que défmi dans l'une quelconque des revendications 1 à 29.

33. Procédé dans lequel on utilise un dispositif de mesure du teint comportant :
- une tête de mesure à appliquer contre la peau, cette tête comportant :
• au moins une fenêtre de sortie (5) par laquelle une lumière est émise,
• au moins une fenêtre de détection (6) pour recevoir la lumière émise par la fenêtre de sortie,
procédé dans lequel on mesure l'atténuation de la lumière dans la peau selon deux trajets de longueurs et/ou d'orientation différentes, sans déplacer le dispositif relativement à la peau.
